(19) **Europäisches Patentamt / European Patent Office / Office européen des brevets**

(11) **EP 2 883 496 B1**

(12) **EUROPEAN PATENT SPECIFICATION**

(45) Date of publication and mention
of the grant of the patent:
**24.08.2016 Bulletin 2016/34**

(51) Int Cl.:
*A61B 5/11* (2006.01)        *A61B 5/00* (2006.01)
*A61H 31/00* (2006.01)

(21) Application number: **13382514.1**

(22) Date of filing: **16.12.2013**

(54) **Device and method for cardiac resuscitation**

Vorrichtung und Verfahren zur kardialen Reanimation

Dispositif et procédé pour réanimation cardiaque

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**17.06.2015 Bulletin 2015/25**

(73) Proprietor: **Osatu, S. Coop.**
**48269 Ermua (ES)**

(72) Inventors:
• **Ruiz Ojeda, Jesus Maria**
  **48196 LEZAMA (ES)**
• **Gonzalez Otero, Digna Maria**
  **48902 BARACALDO (ES)**
• **Ruiz De Gauna Gutierrez, Sofia**
  **48993 GETXO (ES)**
• **Irusta Zarandona, Unai**
  **48014 BILBAO (ES)**
• **Aramendi Ecenarro, Elisabete**
  **20720 AZKOITIA (ES)**

• **Alonso Gonzalez, Erik**
  **48970 BASAURI (ES)**
• **Ayala Fernandez, Unai**
  **48510 TRAPAGARAN (ES)**

(74) Representative: **Igartua, Ismael**
**Galbaian S.Coop.**
**Polo de Innovación Garaia**
**Goiru Kalea 1 - P.O. Box 213**
**20500 Arrasate-Mondragón (ES)**

(56) References cited:
**EP-A2- 2 275 027        WO-A1-2013/109750
WO-A2-2012/065104**

• **SVEN O AASE* ET AL: "Compression Depth
Estimation for CPR Quality Assessment Using
DSP on Accelerometer Signals", IEEE
TRANSACTIONS ON BIOMEDICAL
ENGINEERING, IEEE SERVICE CENTER,
PISCATAWAY, NJ, USA, vol. 49, no. 3, 1 March
2002 (2002-03-01), XP011007210, ISSN:
0018-9294**

EP 2 883 496 B1

**Description**

TECHNICAL FIELD

The present invention is related to devices to assist in performing chest compressions on a patient during cardiopulmonary resuscitation (CPR) or on a manikin during CPR training, and to methods to assist in performing chest compressions on a patient during CPR or on a manikin during CPR training.

PRIOR ART

Systems intended for assisting in the performance of chest compressions on a patient during cardiopulmonary resuscitation (CPR) or on a manikin during a CPR training process are known. The purpose is to provide feedback to the assistant during CPR so that he/she successfully performs chest compressions at a frequency and depth that comply with the recommendations of international cardiopulmonary resuscitation guidelines.

CPR is an important part of the treatment recommended for cardiorespiratory arrest, and it combines chest compressions with mechanical ventilations. Chest compressions are responsible for maintaining minimal blood flow to vital organs. Many scientific publications have demonstrated that in order for chest compressions to be effective it is necessary to perform them with a suitable frequency and depth, a frequency of 100-120 compressions per minute and a depth of 5-6 centimeters being recommended. If not performed correctly, the return of spontaneous circulation probability drops drastically. For this reason, during CPR it is important for the assistant to have a feedback system that guides him/her in performing quality chest compressions.

The conventional way for calculating the depth and frequency of chest compressions consists of using the acceleration signal and obtaining from it the signal of actual chest depth with more or less precision. In most scientific publications and patents in this field, the method proposed for obtaining the signal of actual chest depth consisted of applying the double integration of the acceleration signal. The double integration of the acceleration signal superimposes an unwanted DC offset over the depth signal. To eliminate this component, various alternatives involving the use of reference signals such as force, the application of filters or the detection of the start and end of each compression for applying boundary conditions, have been used. All the proposed alternatives incorporate a certain error. These methods obtain the signal of actual chest depth for each instant and calculate the frequency and depth of each compression from same.

Devices used to assist in performing chest compressions during CPR are known, said devices comprising an accelerometer arranged in a housing which is arranged in a fixed position with respect to the chest of the patient, such that the acceleration signal produced by the accelerometer indicates the movement of the chest of the patient during chest compressions. The housing of the accelerometer is usually in the form of a rigid case which rests on a padded face on the chest of the patient. Said housing can also house other means of the device, such as a processor for processing the acceleration signal, a visual and/or audio system for presenting the results when performing chest compressions, or power supply means of the device, for example.

The known devices calculate the depth and the frequency of chest compressions and report to the assistant the depth of each compression during CPR, which involves providing feedback to the assistant every 0.5-0.6 seconds. On the other hand, these devices are made in the form of a rigid case which is placed between the hands of the assistant and the chest of the patient. Although said devices have areas contacting the chest of the patient that are more or less padded, when performing actual compressions they may cause harm to the patient and strain on the hands of the assistant, therefore limiting the compressions performed by the assistant.

EP1057451A1 describes a device for measuring and using parameters when performing chest compressions on a patient during CPR or on a manikin during CPR training. Said device comprises a compression pad arranged for tracking the movement of the chest of the patient, where the CPR assistant manually exerts force to perform chest compressions, said compression pad comprising an acceleration sensor unit producing acceleration signals which indicate the movement of the chest of the patient during chest compressions, the device also comprising processing means for determining the depth of chest compressions from the acceleration signals received from the acceleration sensor unit by means of double integration.

DISCLOSURE OF THE INVENTION

The object of the invention is to provide a device to assist in performing chest compressions on a patient during CPR or on a manikin during CPR training, and a method to assist in performing chest compressions on a patient during CPR or on a manikin during CPR training, as described in the claims.

One aspect of the invention is related to a device to assist in performing chest compressions on a patient during cardiopulmonary resuscitation (CPR) or on a manikin during CPR training, comprising a compression pad arranged for tracking the movement of the chest of the patient, where the CPR assistant manually exerts force to perform chest compressions, said compression pad comprising an acceleration sensor unit producing acceleration signals which indicate the movement of the chest of the patient or manikin during chest compressions, and the device also comprising processing means for determining the depth of chest compressions from the acceleration signals received from the acceleration sensor unit.

The processing means obtain from said acceleration signals a signal representative of the movement of the chest of the patient, preferably an acceleration signal perpendicular to the chest of the patient, analyze said acceleration signal in the frequency domain in short-time windows, and estimate the mean depth of chest compressions in each window from the mean frequency and the amplitudes and phases of the first harmonics of the signal of each window.

Another aspect of the invention is related to a method to assist in performing chest compressions on a patient during CPR or on a manikin during CPR training, in which a signal representative of the movement of the chest of the patient, preferably an acceleration signal perpendicular to the chest of the patient, is analyzed in the frequency domain in short-time windows, and the mean depth of chest compressions in each window is estimated from the mean frequency and the amplitudes and phases of the first harmonics of the signal of each window.

In the device and the method of the invention, the acceleration signal is analyzed in the frequency domain; the drawbacks derived from calculating the depth by means of the double integration of the acceleration signal are thus prevented, since the problem associated with superimposing a DC component on the depth signal disappears, whereby it is unnecessary to use additional reference signals for eliminating this component. Therefore, since only the acceleration signal is analyzed, a simpler and less expensive solution is obtained. Furthermore, errors that can be produced when the depth signal is calculated by means of double integration are prevented and the DC component is eliminated by means of other reference signals.

On the other hand, known devices report to the CPR assistant the depth of each compression. Taking into account the existing recommendations in terms of the number of compressions to be performed, it means that the assistant obtains a report on how the chest compressions are being performed every 0.5 seconds. By selecting short-time windows for analyzing the acceleration signal and calculating the mean frequency and the mean depth of chest compressions in each window, the device of the invention provides feedback to the assistant once every few seconds with precise data, and without this feedback being a distraction to his/her assistance work.

These and other advantages and features of the invention will become evident in view of the drawings and the detailed description of the invention.

DESCRIPTION OF THE DRAWINGS

Figure 1 shows a schematic view of an assistant performing chest compressions on a patient during CPR with an embodiment of the device of the invention wherein the compression pad and the processing and feedback module are mechanically and electrically coupled to one another forming a single part.

Figure 2 separately shows a schematic perspective view of the compression pad and of the processing and feedback module of the device of Figure 1.

Figure 3 shows a block diagram with the different means that can be included in the compression pad and the processing and feedback module of the device of the invention.

Figure 4 shows a detailed perspective view of the device of Figure 1.

Figure 5 shows the device of Figure 1 with the hands of the assistant placed on the compression pad.

Figure 6 shows a perspective view of a second embodiment of the device wherein the compression pad and the processing and feedback module are mechanically separated from and electrically coupled to one another by means of a cable.

Figure 7 shows the device of Figure 6 with the hands of the assistant placed on the compression pad.

Figure 8 shows the device of Figure 6 with the compression pad between the hands of the assistant.

Figure 9 shows a perspective view of a third embodiment of the device wherein the compression pad and the processing and feedback module are two separate parts that are electrically coupled to one another and mechanically coupled to the hands of the assistant.

Figure 10 shows the device of Figure 9 with the hands of the assistant placed on the compression pad.

Figure 11 shows the device of Figure 9 with the compression pad between the hands of the assistant.

Figure 12 shows a schematic view of the devices of Figures 4, 6 and 9 exchanging information with an external device.

Figure 13 shows a perspective view of a fourth embodiment of the device wherein the compression pad and the processing and feedback module are mechanically separated from and electrically coupled to one another by means of a cable, the processing and feedback module being incorporated in an external device.

Figure 14 shows a block diagram of an embodiment of the method of the invention for assisting in the application of chest compressions during CPR.

Figure 15a shows the acceleration signal perpendicular to the chest of the patient or manikin in a window.

Figure 15b shows the spectrum of the acceleration signal in the window of Figure 15a.

Figure 15c shows the signal of the actual depth of chest compressions by means of a continuous line, and the periodic dummy signal of mean depth by means of a dotted line, both signals without a DC component.

DETAILED DISCLOSURE OF THE INVENTION

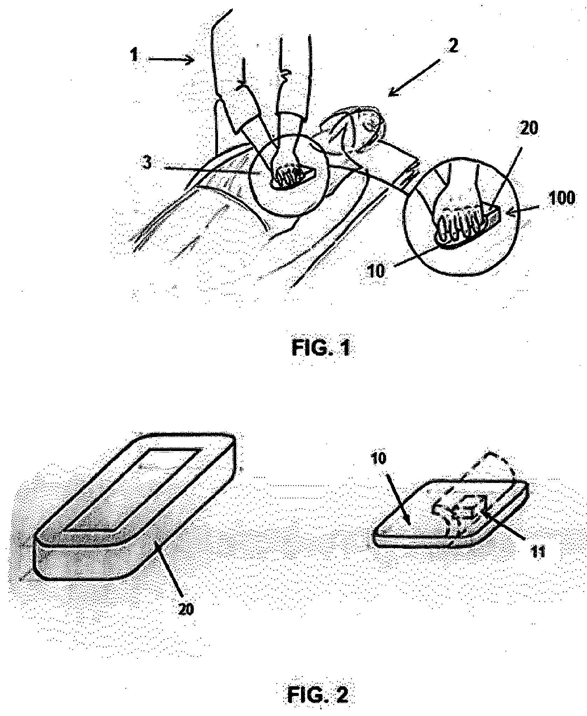

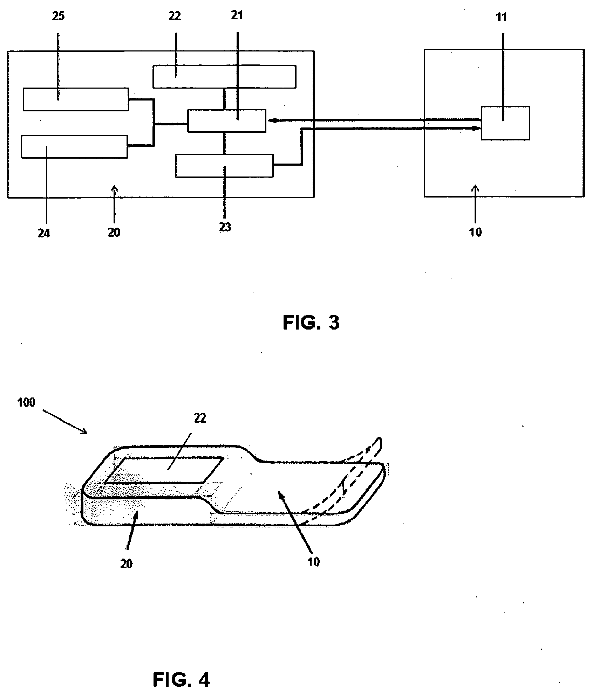

Figure 1 shows a schematic view of an assistant 1 performing chest compressions on a patient or manikin 2 during CPR with an embodiment of the device 100 of the invention. The device 100 of the invention comprises an acceleration sensor unit 11 producing acceleration signals indicating the movement of the chest 3 of the patient or manikin 2 during chest compressions, and processing means 21, schematically shown in the block diagram of Figure 3, obtaining from said acceleration signals a signal representative of the movement of the chest of the patient 2, preferably an acceleration signal perpendicular to the chest 3 of the patient 2. Said processing means 21 analyze said signal in the frequency domain in short-time windows 60 and estimate the mean depth $s_m$ of chest compressions in each window 60 from the mean frequency $f_{cc}$ and the amplitudes and phases of the first harmonics of the acceleration signal of each window 60. The amplitudes and phases of the first harmonics corresponding to the depth signal are calculated from the mean frequency $f_{cc}$ and from the amplitudes and phases of the first harmonics of the acceleration, and a mean depth value for the entire window 60 is estimated from the amplitudes and phases of the first harmonics corresponding to the depth signal.
The device 100 comprises two parts. The first part of the device 100 is a compression pad 10 which is arranged on the chest 3 of the patient 2 and allows tracking the movement of the chest 3 of the patient 2 when the assistant performs the chest compressions and the chest 3 moves. This compression pad 10 is a thin and flexible support, such as a computer mouse pad, for example, which allows bending and/or twisting, and on which the CPR assistant 1 exerts force with his/her hands to perform chest compressions. This compression pad 10 is made of a flexible material such as foam rubber or silicone rubber, for example. The thin and flexible characteristics of the compression pad 10 do not cause harm on the chest 3 of the patient 2, or cause discomfort or strain on the hands of the assistant 1. The device 100 comprises a second portion which is a processing and feedback module 20 which in this embodiment is a rigid and non-deformable part. The compression pad 10 and the processing and feedback module 20 are electrically coupled to one another and in this first embodiment, they are also mechanically coupled to one another.
Figure 2 separately shows a schematic perspective view of the compression pad 10 and of the processing and feedback module 20 of the device 100 of Figure 1. The compression pad 10 internally comprises the acceleration sensor unit 11 embedded therein, and it produces acceleration signals indicating the movement of the chest 3 of the patient 2 during chest compressions. In a preferred embodiment, the acceleration sensor unit 11 is the only element of the device 100 in the compression pad 10, whereby there are no additional elements that compromise the flexibility of said compression pad 10.
Figure 3 shows a block diagram with the different means that can be included in the compression pad 10 and the processing and feedback module 20 of the device 100 of Figure 1 and their interconnection. The processing and feedback module 20 comprises processing means 21 for determining a mean frequency $f_{cc}$ and a mean depth $s_m$ of chest compressions from the acceleration signals received from the acceleration sensor unit 11. The processing and feedback module 20 also comprises feedback means 22 for providing feedback to the CPR assistant 1 on the deviations of the mean depth $s_m$ and mean frequency $f_{cc}$ of chest compressions performed with respect to recommended reference values. Said feedback means 22 can be visual means, such as a display or a plurality of LEDs, for example, and/or audio means which allow the assistant 1 to hear the deviations being produced. The processing and feedback module 20 also comprises power supply means 23 for powering the different means of the device 100, the compression pad 10 and the processing and feedback module 20 being electrically connected in any of the embodiments of the device 100.

The acceleration sensor unit 11 can be a single-, dual- or tri-axis accelerometer, or there can be one, two or three single-axis accelerometers arranged orthogonal to one another, the processing means 21 determining an acceleration signal perpendicular to the chest 3 of the patient or manikin 2 as a combination of the acceleration signals produced by the acceleration sensor unit 11. The processing means 21 of the processing and feedback module 20 receive the acceleration signals from the acceleration sensor unit 11 and calculate a single acceleration signal perpendicular to the chest 3 of the patient 2. Due to the small size of the accelerometers that are embedded in the compression pad 10 and despite the thinness thereof, said compression pad 10 maintains its flexibility characteristics.

As indicated above, once the processing means 21 have calculated the acceleration signal perpendicular to the chest 3 of the patient 2, said means analyze the acceleration signal in the frequency domain in windows 60 having a duration of a few seconds when performing chest compressions. The processing means 21 calculate the value of the mean frequency $f_{cc}$ of chest compressions or fundamental frequency, and the amplitude $A_i$ and phase $\theta_i$ of the first harmonics of the acceleration signal in each window 60 by means of spectral analysis. Once the values of $A_i$ and $\theta_i$ are known, the processing means 21 calculate the amplitude $S_i$ and phase $\psi_i$ of the first harmonics of the depth signal by means of the following formulas:

$$S_i = 1000 * Ai/(2\pi * i * fcc)^2, \text{ and}$$

$$\Psi_i = \theta_i + \pi$$

for a number of harmonics i = 1, 2,..,N representative of the acceleration signal and of the depth signal in each window 60. Finally, the processing means 21 estimate the value of the mean depth $s_m$ of chest compressions in each window 60 from parameters $S_i$ and $\psi_i$ using an estimator.

The processing and feedback module 20 can also comprise data storage means 24 for saving the calculated values of the mean depth $s_m$ and mean frequency $f_{cc}$ of chest compressions in each selected window 60 and the parameters $A_i$, $\theta_i$, $S_i$ and $\psi_i$ calculated during the calculation process by the processing means 21. These data storage means 24 can be fixed or removable means.

The processing and feedback module 20 can also comprise communication means 25 for exchanging information with external devices, such as an automated external defibrillator (AED), a monitor-defibrillator, or a computer, for example, in a wired or wireless manner, previously receiving the information from the processing means 21.

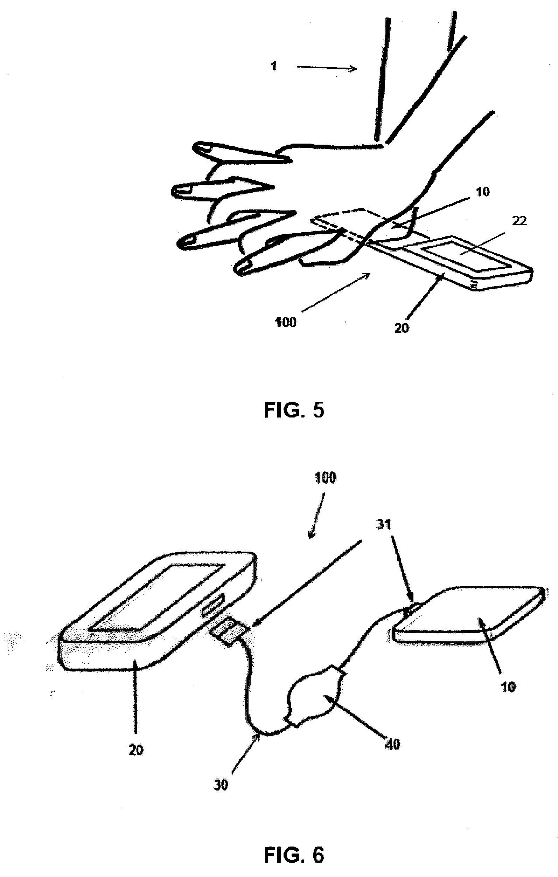

Figure 4 shows a detailed perspective view of the embodiment of the device 100 of Figure 1. In said embodiment, the compression pad 10 and the processing and feedback module 20 are mechanically coupled to one another forming a one-piece compact device. Figure 5 shows the device 100 of Figure 4 in a position which allows placing said device 100 directly on the chest 3 of the patient 2, such that the assistant 1 exerts force with his/her hands placed on the compression pad 10 and leaves the processing and feedback module 20 free. In another arrangement (not shown in the drawings), the compression pad 10 can be arranged between the hands of the assistant 1 in the position deemed by said assistant 1 as most suitable for exerting force and for leaving the processing and feedback module 20 free, and thus being able to view and/or listen to the indications emitted by the feedback means 22. In any case, the assistant 1 can place the device 100 in different positions during CPR.

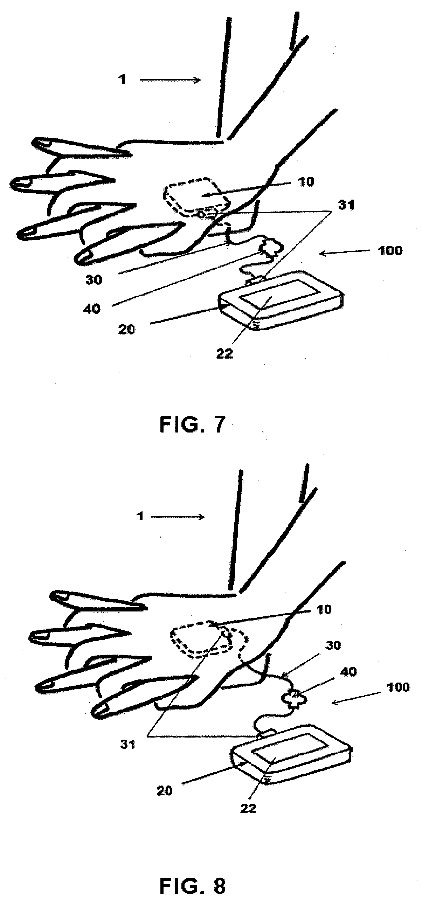

Figure 6 shows a perspective view of a second embodiment of the device 100, wherein the compression pad 10 and the processing and feedback module 20 are mechanically separated from and electrically connected to one another by means of a cable 30, thus configuring the device 100 in two parts. The cable 30 which allows electrical connection comprises a connector 31 at each end, said connectors 31 being connected respectively to the compression pad 10 and to the processing and feedback module 20. The cable 30 comprises a collector reel 40 which allows placing the processing and feedback module 20 in different positions, such that the assistant 1 can place said module in the position that best suits him/her. Figure 7 shows the device 100 of Figure 6 in a position which allows placing the compression pad 10 directly on the chest 3 of the patient 2, such that the assistant 1 exerts force with his/her hands placed on the compression pad 10, and the processing and feedback module 20 can be placed in any position allowed by the cable 30. Figure 8 shows the device 100 of Figure 6 with the compression pad 10 between the hands of the assistant 1 such that the assistant 1 exerts force on the compression pad 10 with one hand and the other hand rests on the chest 3 of the patient 2, transmitting the exerted force to the patient. The processing and feedback module 20 can therefore be placed in any position allowed by the cable 30.

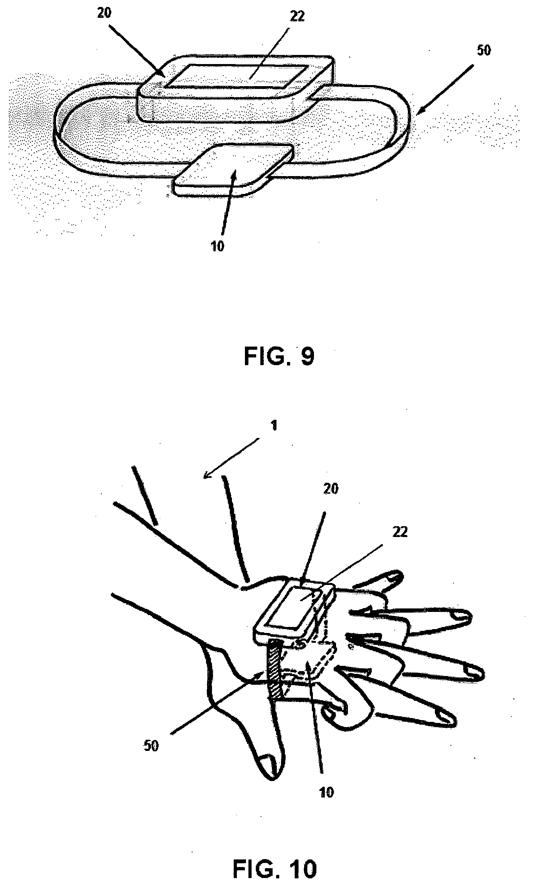

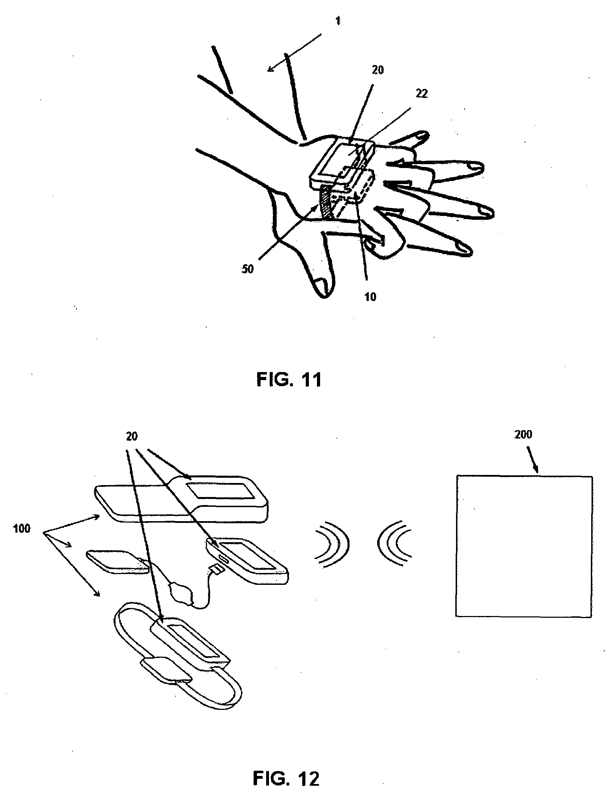

Figure 9 shows a perspective view of a third embodiment of the device 100, wherein the compression pad 10 and the processing and feedback module 20 are two separate parts that are electrically coupled to one another and mechanically coupled to the hands of the assistant 1. The compression pad 10 and the processing and feedback module 20 are mechanically coupled to one another by fixing means 50, said fixing means 50 allowing fixing the processing and feedback module 20 to the back of one of the hands of the assistant 1, and the fixing means 50 have at the other end the compression pad 10 that can be placed on the chest 3 of the patient 2 or between the hands of the assistant 1. These fixing means 50 may or may not be elastic, having the shape of a band. The fixing means 50 allow the electrical connection between the compression pad 10 and the processing and feedback module 20, the band forming the fixing means 50 internally comprising an electric cable (not shown in the drawings). Figure 10 shows the device 100 of Figure 9 in a position which allows placing the compression pad 10 directly on the chest 3 of the patient 2. The processing and feedback module 20 is placed at the back of one hand, the hands are clasped together, and the band forming the fixing means 50 is slipped around the hands placing the compression pad 10 in the lower portion of the second hand, such that said compression pad 10 can be arranged directly on the chest 3 of the patient 2. Therefore, the assistant 1 exerts force with his/her hands placed on the compression pad 10 when performing chest compressions. Figure 11 shows the device 100 of Figure 9 with the compression pad 10 between the hands of the assistant 1. The processing and feedback module 20 is placed at the back of one hand, and the band forming the fixing means 50 is slipped around said hand, placing the compression pad 10 in the lower portion of the first hand, such that the assistant 1 exerts force on the compression pad 10 with his/her first hand, said first hand resting on the second hand, and the second hand resting on the chest 3 of the patient 2, transmitting the exerted force to the patient.

As shown Figure 12, the embodiments of the devices 100 shown in Figures 4, 6 and 9 enable the communication means 25 (not shown in the drawings) arranged in the processing and feedback module 20 to exchange information with an external device 200, such as an AED, a monitor-defibrillator, a computer, a tablet, a smartphone, or any other device, for example, in a wireless manner.

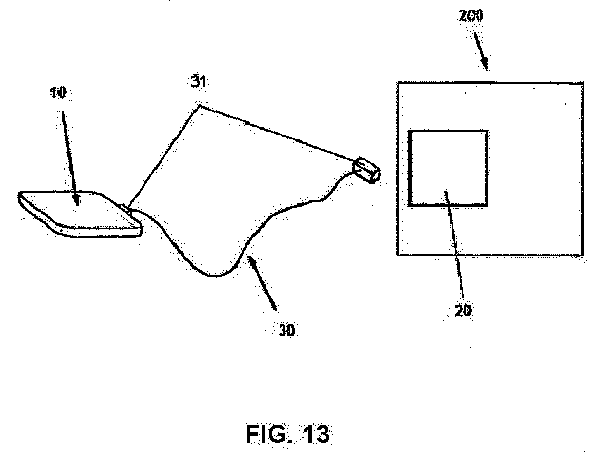

Figure 13 shows a perspective view of a fourth embodiment of the device 100, wherein the compression pad 10 and the processing and feedback module 20 are mechanically separated from and electrically coupled to one another by means of a cable 30, the processing and feedback module 20 being incorporated in an external device 200. Said external device 200 can be an AED, a monitor-defibrillator, or a computer wherein the processing and feedback module 20 can be housed with its corresponding means. The cable 30 allowing electrical connection comprises a connector 31 at each end, said connectors 31 being connected respectively to the compression pad 10 and to the processing and feedback module 20 which is located in the device external 200. The compression pad 10 can be placed directly on the chest 3 of the patient 2 (not shown in the drawings), such that the assistant 1 exerts force with his/her hands placed on the compression pad 10, or the compression pad 10 can be placed between the hands of the assistant 1 such that the assistant 1 exerts force on the compression pad 10 with one hand and the other hand rests on the chest 3 of the patient 2, transmitting the exerted force to the patient. The processing and feedback module 20 which is located in the external device 200 can be placed in any position allowed by the cable 30.

None of the devices 100 shown has to be used by the assistant 1 in a fixed position with respect to the chest 3 of the patient 2. The assistant 1 can choose the position he/she deems most comfortable. The arrangement of the device 100 can be changed if desired while performing CPR.

In the method of the invention, a signal representative of the movement of the chest 3 of the patient 2, preferably an acceleration signal perpendicular to the chest 3 of the patient 2, is analyzed in the frequency domain in short-time windows 60, and the mean depth $s_m$ of chest compressions in each window 60 is estimated from the mean frequency $f_{cc}$ and the amplitudes and phases of the first harmonics of the signal of each window 60.

Chest compressions performed by the assistant on the patient must be performed according to the recommendations of resuscitation guidelines to obtain quality chest compressions. Chest compressions are basically a repetitive action of compression-relaxation of the chest of the patient. For this reason, the depth and acceleration signals associated with each compression during two consecutive compressions are very similar to one another. This means that the depth and acceleration signals seen through a short-time window can be considered as periodic signals.

The theory of Fourier series development of periodic signals proposes decomposing a periodic signal into the sum of infinite sinusoids of integral multiples of fundamental frequency or harmonics. Therefore, considering the periodicity of the depth and acceleration signal seen through a short-time window, these signals could be analytically described in the following manner for the duration of that window:

Acceleration:

$$a(t) = \sum_{i=1}^{N} A_i \cos(2\pi i f_{cc} t + \theta_i) \qquad (1)$$

Depth:

$$s(t) = \sum_{i=1}^{N} S_i \cos(2\pi i f_{cc} t + \Psi_i) \qquad (2)$$

where:

a(t) represents the acceleration signal perpendicular to the chest of the patient in meters/second$^2$(m/s$^2$) during the studied window without a DC component.

s(t) represents the depth signal in millimeters (mm) during the studied window without a DC component.

$f_{cc}$ is the value of the fundamental frequency of the acceleration and depth which corresponds with the value of the mean frequency of chest compressions in Hertz (Hz).

In theory, N should be infinite. In the method of the invention N can take small values without compromising precision, from N=1 to a value of N=3 or 4 or a similar integer value.

$A_i$ and $\theta_i$ are the amplitude in m/s$^2$ and the phase of the harmonic of the acceleration of the order i in radians, respectively.

$S_i$ and $\psi_i$ are the amplitude in mm and the phase of the harmonic of the depth of the order i in radians, respectively.

Acceleration is the second derivative of movement over time. By deriving the depth equation (2) twice over time and comparing the result with the acceleration equation (1), relationships between the amplitudes and harmonic phases of acceleration and depth are obtained:

$$S_i = 1000 \frac{A_i}{(2\pi i f_{cc})^2} \qquad i = 1,2,3 \dots N \qquad (3)$$

$$\Psi_i = \theta_i + \pi \qquad i = 1,2,3 \dots N \qquad (4)$$

The method of the invention is implemented, for example, in a device 100 such as that shown the Figures 1, 6, 9 and 13, suitable for assisting in performing chest compressions on a patient or manikin 2 during cardiopulmonary resuscitation (CPR). Said device 100 comprises a compression pad 10 where the CPR assistant 1 manually exerts force to perform chest compressions. Said compression pad 10 internally comprises an acceleration sensor unit 11, said acceleration sensor unit 11 comprising a single-, dual- or tri-axis accelerometer, or one, two or three single-axis accelerometers arranged orthogonal to one another, producing acceleration signals indicating the movement of the chest 3 of the patient 2 during chest compressions. The device 100 also comprises processing means 21 for processing the acceleration signals received from the acceleration sensor unit 11, and feedback means 22 for providing feedback to the assistant 1.

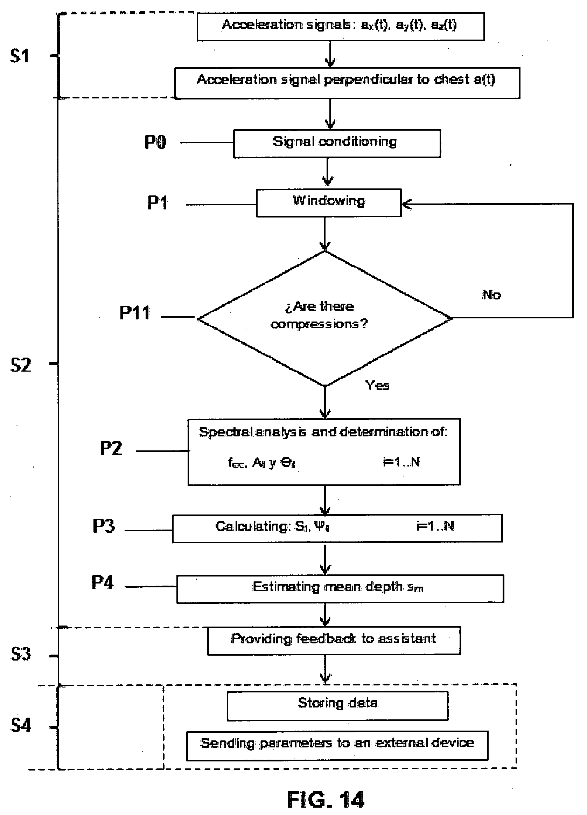

Figure 14 shows a block diagram of an embodiment of the method of the invention to assist in CPR. Said method comprises:

- a chest compression step S1 for compressing the chest of the patient, wherein the processing means 21 receive the acceleration signals from the acceleration sensor unit 11 and determine an acceleration signal perpendicular to the chest 3 of the patient or manikin 2 as a combination of the acceleration signals produced by the acceleration sensor unit 11,

- a processing step S2 for processing the acceleration signal, wherein the processing means 21 calculate the value of the mean frequency $f_{cc}$ and of the mean depth $s_m$ of chest compressions in each selected window 60, and

- a feedback step S3 for providing feedback to the assistant 1, wherein the feedback means 22 provide feedback to the assistant 1 the deviations of the mean depth $s_m$ and the frequency $f_{cc}$ of chest compressions with respect to recommended reference values.

The processing step S2 comprises:

- an optional signal conditioning phase P0 in which the acceleration signal perpendicular to the chest 3 of the patient or manikin 2 received by the processing means 21 is conditioned by means of a filter or an equivalent system eliminating the DC component or low frequency components of the acceleration signal,

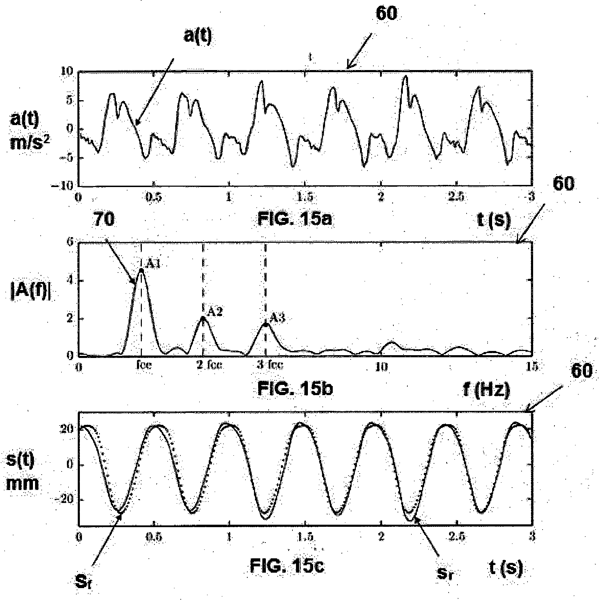

- a windowing phase P1 for windowing the acceleration signal vertical and perpendicular to the chest 3 of the patient or manikin 2 after the signal conditioning phase P0, the processing means 21 selecting short-time windows 60, for example of 3 or 4 seconds, for the acceleration signal received and to be treated. Windows known in the state of the art such as the rectangular, triangular, Bartlett, Hanning, or Hamming windows, or any other windows with properties similar to those mentioned, can be used. The already windowed acceleration signal can also be conditioned by subtracting the DC component from said signal, for example. Figure 15a shows the acceleration signal a(t) in $m/s^2$, corresponding to the acceleration signal perpendicular to the chest 3 of the patient or manikin 2 in a three-seconds window,

- an optional verification phase P11 for verifying the existence of chest compressions after the windowing phase P1, wherein the processing means 21 analyze the acceleration signal for verifying the existence of chest compressions in each window 60. To that end, the waveform of the acceleration signal, its energy, mean power or any other parameter indicating the presence or absence of compressions, can be analyzed. The decision to continue with the processing step S2 for processing the acceleration signal is made if chest compressions take place in the window 60. If chest compressions do not take place in said window 60, the arrival of the following window 60 of the acceleration signal is waited for, and the verification phase P11 for verifying the existence of chest compressions is restarted. This phase is optional because the presence of chest compressions can also be verified in the frequency domain by means of spectral analysis of the window 60 of the acceleration signal,

- a spectral analysis phase P2 in which the processing means 21 perform a spectral analysis of the windows 60 of the acceleration signal in the frequency domain. In order to obtain a spectrum 70 of the window 60 of the acceleration signal, Fast Fourier Transform (FFT) which allows obtaining the spectrum in a computationally efficient manner is preferably used, however Discrete Fourier Transform (DFT) or any other time-frequency transform that allows analyzing the window 60 of the acceleration signal in the frequency domain could also be used. Any estimator of the power spectral density of the window 60 of the acceleration signal, which is either conventional (Periodogram, Bartlett, Welch, etc.) or parametric, could also be used. By means of analyzing the spectrum 70 of the window 60 of the acceleration signal, the modulus of the FFT preferably scaled in $m/s^2$, the fundamental component of the spectrum 70 is detected. The frequency at which it occurs provides the fundamental frequency or value of mean frequency $f_{cc}$ of compressions in Hz or compressions per seconds. If it is multiplied by 60 the mean frequency of compressions is obtained in compressions per minute (cpm). The value of the modulus of the FFT in that frequency $f_{cc}$ provides the fundamental component or the first harmonic of acceleration $A_1$, and the phase of the FFT at that frequency $f_{cc}$ provides the phase of the fundamental component or first harmonic $\theta_1$. The positive peak at about $2f_{cc}$ of the modulus of the FFT provides $A_2$, and the phase of the FFT at the frequency $2f_{cc}$ of this peak provides $\theta_2$. With this system, $A_i$ and $\theta_i$ for i=1, 2...N are calculated, where N is the number of harmonics of the acceleration considered. It can be considered that the three first harmonics are representative of the spectrum 70 of the acceleration signal. Figure 15b shows the spectrum 70 of the window 60 of the acceleration signal, said spectrum 70 being evaluated by means of a 1024 point FFT using zero padding. The modulus $|A(f)|$ of the values provided by the FFT scaled in amplitudes in $m/s^2$ is shown. Figure 15b also indicates the fundamental frequency $f_{cc}$ of compressions and the amplitude of the three first harmonics $A_1$, $A_2$ and $A_3$. Phases $\theta_1$, $\theta_2$ and $\theta_3$ would be obtained from the $|A(f)|$ phase, which is not depicted,

- a calculation phase P3 for calculating values of the depth spectrum parameters for the depth of chest compressions in each selected window 60 after the spectral analysis phase P2. Once the values of $f_{cc}$, $A_i$ and $\theta_i$ for i=1, 2...N of the analyzed window 60 are known, the processing means 21 calculate the values of the depth spectrum parameters $S_i$ and $\psi_i$, which are the modulus of the amplitude and the phase of the harmonic of the depth of the order i, respectively, by means of formulas (3) and (4):

$$S_i = 1000 \cdot A_i / (2\pi \cdot i \cdot f_{cc})^2 \,,$$

and

$$\Psi_i = \theta i + \pi$$

for a number of harmonics i = 1, 2,...,N representative of the depth, and

- an estimation phase P4 for estimating the mean depth $s_m$ of chest compressions of each window 60 after the calculation phase P3 for calculating the values of the depth spectrum parameters, wherein the processing means 21 calculate the value of the mean depth $s_m$ of chest compressions during the window 60, from the values of parameters $S_i$ and $\psi_i$, using an estimator. Different estimators may be valid, but their precision may not be suitable to subsequently provide information to the assistant 1. For example, if only parameters $S_1$ and $\psi_1$ corresponding to the first harmonic are calculated, the mean depth $s_m$ could be estimated as $2A_1$. If several harmonics are calculated, another alternative can consist of applying formula (2) for the time instants in which the fundamental harmonic is maximum and minimum. Subtracting both values would give another possible estimator for depth. Another alternative is to reconstruct, by means of formula (2), a dummy signal $s_f$ representing the depth of an average chest compression cycle for the window 60, and which is therefore calculated for a period spanning from t=0 to $t=1/f_{cc}$. The difference between the maximum and the minimum of this signal provides another estimator for the mean depth $s_m$ of the analyzed window 60. Figure 15c shows, by means of a continuous line, the signal of actual depth $s_r$ without a DC component, and by means of a dotted line, the periodic dummy signal $s_f$ of mean depth analytically reconstructed by means of formula (2).

Once the values of mean frequency $f_{cc}$ and mean depth $s_m$ are known, they are compared in the feedback step S3 with the reference values recommended by resuscitation guidelines, the information to be provided to the assistant 1 visually or by way of audio by the feedback means 22 being obtained.

The device 100 can also comprise data storage means 24 for saving all or some of the calculated values of the mean depth $s_m$ and the mean frequency $f_{cc}$ of chest compressions in each selected window 60 and the parameters $A_i$, $\theta_i$, $S_i$ and $\psi_i$ calculated during the calculation process by the processing means 21. The device 100 can also comprise communication means 25 for exchanging information in a wired or wireless manner with an external device 200, such as an AED, a monitor-defibrillator or a computer, for example. The method can also comprise:

- a data storage and communication step S4, after the feedback step S3, wherein the values of the mean frequency $f_{cc}$ and the mean depth $s_m$ of chest compressions, as well as the values of parameters $A_i$, $\theta_i$, $S_i$, and $\psi_i$ of a number of harmonics i = 1, 2, ..., N, of each selected window 60, are saved in the data storage means 24 and/or sent to an external device 200 by means of the communication means 25.

The depth and acceleration signals for the time window 60, which are reconstructed by means of formulas (1) and (2) respectively from said parameters, will not exactly coincide with the true acceleration and depth signals, but they will be similar. They provide a representation of the depth of the average compression cycle for that window 60, which does not contemplate the differences between the different cycles of the window 60. The method is performed for the successive windows 60 of the analysis. The windows 60 can correspond with adjacent time intervals or can overlap in time. Therefore, for example, windows having a duration of 4 seconds can be used, but information may be provided to the assistant 1 once every two seconds if a two-seconds overlap is used (in absolute value, each window only advances two seconds although the duration thereof is 4 seconds).

The method of the invention contemplates all the possible alternatives for estimating the mean frequency $f_{cc}$ and the mean value of the depth $s_m$ of chest compressions for a short-time window 60 working in the frequency domain. Therefore, it also takes into account the alternative of starting from acceleration signals, obtaining a speed signal perpendicular to the chest 3 of the patient 2 and processing said speed signal in the frequency domain.

In said alternative, the steps of the method are the same as in the embodiment of the method described above, taking into account that an integration phase P12 (not shown in the drawings) for integrating the acceleration signal perpendicular to the chest 3 of the patient or manikin 2, is added in the processing step S2 before the spectral analysis phase P2 and after the verification phase P11 for verifying the existence of chest compressions. In said phase P12, the acceleration signal is integrated once and the speed signal in each window 60 is calculated, performing in the spectral analysis phase P2 an analysis of the windows 60 of the speed signal spectrum. The mean frequency $f_{cc}$ of chest compressions in each selected window 60 is calculated, and parameters $V_i$ and $\varphi_i$, which are the modulus of the amplitude and the phase of the harmonic of the speed signal of the order i, respectively, of each window (60) for a number of harmonics i = 1, 2,...,N, representative of the speed signal frequency spectrum. In the calculation phase P3 for calculating the values of depth parameters, the values of depth parameters $S_i$ and $\psi_i$ are calculated by means of formulas:

$$S_i = 1000 \cdot V_i / (2\pi \cdot i \cdot f_{cc}) \qquad (5),$$

and

$$\Psi_i = \varphi_i - \pi/2 \qquad (6)$$

for a number of harmonics i = 1, 2,...,N representative of the depth.

**Claims**

1. Device to assist in performing chest compressions on a patient or manikin (2) during cardiopulmonary resuscitation (CPR) comprising a compression pad (10) arranged for tracking the movement of the chest (3) of the patient or manikin (2), where the CPR assistant (1) manually exerts force to perform chest compressions, said compression pad (10) comprising an acceleration sensor unit (11) producing acceleration signals representative of the movement of the chest (3) of the patient or manikin (2) during chest compressions, and the device (100) also comprising processing means (21) for determining the depth of chest compressions from the acceleration signals received from the acceleration sensor unit (11), **characterized in that** the processing means (21) obtain a signal representative of the movement of the chest (3) of the patient or manikin (2), preferably an acceleration signal perpendicular to the chest (3) of the patient or manikin (2), from said acceleration signals, analyze said signal in the frequency domain in short-time windows (60) and estimate the mean depth ($s_m$) of chest compressions in each window (60) from the mean frequency ($f_{cc}$) and the amplitudes and phases of the first harmonics of the signal of each window (60).

2. Device according to the preceding claim, wherein the acceleration sensor unit (11) is a single-, dual- or tri-axis accelerometer, or one, two or three single-axis accelerometers arranged orthogonal to one another, the processing means (21) determining the acceleration signal perpendicular to the chest (3) of the patient or manikin (2) as a combination of the acceleration signals produced by the acceleration sensor unit (11).

3. Device according to claim 1 or 2, wherein the compression pad (10) is a flexible support which allows bending and/or twisting, the acceleration sensor unit (11) being arranged inside said compression pad (10), said acceleration sensor unit (11) preferably being the only element of the device (100) arranged in the compression pad (10).

4. Device according to any of claims 1 to 3 comprising a processing and feedback module (20) wherein there are arranged the processing means (21), feedback means (22) for providing feedback to the CPR assistant, visually or by way of audio, about the deviations of the mean depth ($s_m$) and mean frequency ($f_{cc}$) of chest compressions with respect to recommended reference values, and power supply means (23) for powering the different means of the device (100), the compression pad (10) and the processing and feedback module (20) being electrically connected.

5. Device according to claim 4, wherein the processing and feedback module (20) comprises data storage means (24) for saving the values of the mean depth ($s_m$) and mean frequency ($f_{cc}$) of chest compressions and the parameters calculated during the analysis of the acceleration signals in the frequency domain by the processing means (21), these data storage means (24) being fixed or removable.

6. Device according to claim 4 or 5, wherein the processing and feedback module (20) comprises communication means (25) for exchanging information with external devices in a wired or wireless manner.

7. Device according to any of claims 4 to 6, wherein the compression pad (10) and the processing and feedback module (20) are mechanically coupled to one another, the device (100) being placed on the chest (3) of the patient or manikin (2) with the hands of the assistant (1) placed on the compression pad (10), or the compression pad (10) being located between the hands of the assistant (1).

8. Device according to any of claims 4 to 6, wherein the compression pad (10) and the processing and feedback module (20) are electrically connected by means of a cable (30) with connectors (31) at each of the ends thereof, said cable (30) having a collector reel (40) which allows placing the processing and feedback module (20) in different positions, the compression pad (10) being placed on the chest (3) of the patient or manikin (2) with the hands of the assistant (1) placed on the compression pad (10), or the compression pad (10) being placed between the hands of the assistant (1).

9.  Device according to any of claims 4 to 6, wherein the compression pad (10) and the processing and feedback module (20) are mechanically coupled to one another by fixing means (50) which allow securing the processing and feedback module (20) to the back of one of the hands of the assistant (1), said fixing means (50) allowing the electrical connection between the compression pad (10) and the processing and feedback module (20), the compression pad (10) being placed on the chest (3) of the patient or manikin (2) with the hands of the assistant (1) placed on the compression pad (10), or the compression pad (10) being placed between the hands of the assistant (1).

10. Device according to any of claims 4 to 6, wherein the compression pad (10) and the processing and feedback module (20) are electrically connected by means of a cable (30) with connectors (31), the processing and feedback module (20) being incorporated in an external device (200), and the compression pad (10) being placed on the chest (3) of the patient or manikin (2) with the hands of the assistant (1) placed on the compression pad (10), or the compression pad (10) being placed between the hands of the assistant (1).

11. Method to assist in performing chest compressions on a patient or manikin (2) during cardiopulmonary resuscitation (CPR), **characterized in that** a signal representative of the movement of the chest (3) of the patient or manikin (2), preferably an acceleration signal perpendicular to the chest (3) of the patient or manikin (2), is analyzed in the frequency domain in short-time windows (60), and the mean depth ($s_m$) of chest compressions in each window (60) is estimated from the mean frequency ($f_{cc}$) and the amplitudes and phases of the first harmonics of the signal of each window (60).

12. Method according to claim 11, implemented in a device (100) suitable for assisting in performing chest compressions on a patient or manikin (2) during CPR, comprising a compression pad (10) where the CPR assistant (1) manually exerts force to perform chest compressions, said compression pad (10) internally comprising an acceleration sensor unit (11) producing acceleration signals indicating the movement of the chest (3) of the patient or manikin (2) during chest compressions, processing means (21) for processing the acceleration signals received from the acceleration sensor unit (11), and feedback means (22) for providing feedback to the assistant (1), said method comprising:

    - a chest compression step (S1) for compressing the chest of the patient, wherein the processing means (21) receive the acceleration signals from the acceleration sensor unit (11) and determine an acceleration signal perpendicular to the chest (3) of the patient or manikin (2) as a combination of the acceleration signals produced by the acceleration sensor unit (11),
    - a processing step (S2) for processing the acceleration signal perpendicular to the chest (3) of the patient or manikin (2), wherein the processing means (21) calculate the value of the mean frequency ($f_{cc}$) and of the mean depth($s_m$) of chest compressions in each selected window (60), and
    - a feedback step (S3) for providing feedback to the assistant (1), wherein the feedback means (22) provide feedback to the assistant (1) the deviations of the mean depth ($s_m$) and the frequency ($f_{cc}$) of chest compressions with respect to recommended reference values.

13. Method according to claim 12, wherein the processing step (S2) comprises:

    - a windowing phase (P1) for windowing the acceleration signal perpendicular to the chest (3) of the patient or manikin (2), the processing means (21) selecting the short-time windows (60) for the acceleration signal received,
    - a spectral analysis phase (P2) after the windowing phase (P1), the processing means (21) conducting a spectral analysis of the windows (60) of the acceleration signal in the frequency domain, calculating the frequency spectrum (70) of the acceleration signal by means of a frequency estimator, calculating in the frequency spectrum (70) of the acceleration signal the fundamental frequency or value of the mean frequency ($f_{cc}$) of chest compressions in each selected window (60), and the parameters $A_i$ and $\theta_i$, which are the value of the amplitude and the phase of the harmonic of the frequency spectrum of the acceleration signal of the order i, respectively, of each window (60) for a number of harmonics i = 1, 2,...,N, representative of the frequency spectrum (70),
    - a calculation phase (P3) for calculating the values of the depth parameters of each selected window (60) after the spectral analysis phase (P2), the processing means (21) calculating the values of the depth parameters $S_i$ and $\psi_i$, which are the value of the amplitude and the phase of the harmonic of the depth spectrum of the order i, respectively, by means of formulas

$$S_i = 1000*Ai/(2\pi*i*fcc)^2,$$

and

$$\Psi_i = \theta_i + \pi$$

for a number of harmonics i = 1, 2,...,N representative of the depth spectrum, and
- an estimation phase (P4) for estimating the mean depth ($s_m$) of chest compressions of each window (60) after the calculation phase (P3) for calculating the values of the depth parameters, wherein the processing means (21) calculate the value of the mean depth ($s_m$) from the values of parameters $S_i$ and $\psi\theta_i$, using an estimator.

14. Method according to claim 12 or 13, wherein the device (100) comprises data storage means (24) and communication means (25), the method comprising a data storage and communication step (S4) after the reporting step (S3), wherein the values of the mean frequency ($f_{cc}$) and the mean depth ($s_m$) of chest compressions, as well as the values of parameters $A_i$, $\theta_i$, $S_i$, and $\psi_i$ of a number of harmonics i = 1, 2, ..., N, of each selected window (60), are saved in the data storage means (24) and/or sent to an external device (200) by means of the communication means (25).

15. Method according to claim 13 or 14, comprising in the processing step (S2) a signal conditioning phase (P0) before the windowing phase (P1), in which the acceleration signal perpendicular to the chest (3) of the patient or manikin (2) received by the processing means (21) is conditioned by means of a filtering system eliminating the DC component or low frequency components of the acceleration signal, and a verification phase (P11) for verifying the existence of chest compressions after the windowing phase (P1), wherein the acceleration signal is analyzed for verifying the existence of chest compressions in each window (60), and the decision to continue with the spectral analysis phase is made if chest compressions take place in the window (60), or if chest compressions do not take place in the window (60), the arrival of the following window (60) of the acceleration signal is waited for, and the verification phase (P11) for verifying the existence of chest compressions is restarted.

**Patentansprüche**

1. Vorrichtung zum Unterstützen der Durchführung der Brustkompressionen an einem Patienten oder Modell (2) während der Herz-Lungen-Wiederbelebung (HLW), die ein Kompressionskissen (10) umfasst, das so ausgelegt ist, dass es die Bewegung der Brust (3) des Patienten oder Modells (2) verfolgt, wobei der HLW-Assistent (1) manuell Kraft ausübt, um Brustkompressionen durchzuführen, wobei das Kompressionskissen (10) eine Beschleunigungssensoreinheit (11) umfasst, die Beschleunigungssignale erzeugt, die für die Bewegung der Brust (3) des Patienten oder Modells (2) während Brustkompressionen repräsentativ sind, und wobei die Vorrichtung (100) außerdem ein Verarbeitungsmittel (21) zum Ermitteln der Tiefe von Brustkompressionen anhand der von der Beschleunigungssensoreinheit (11) empfangenen Beschleunigungssignale umfasst, **dadurch gekennzeichnet, dass** das Verarbeitungsmittel (21) ein Signal, das für die Bewegung der Brust (3) des Patienten oder Modells (2) repräsentativ ist, vorzugsweise ein Beschleunigungssignal senkrecht zur Brust (3) des Patienten oder Modells (2), aus den Beschleunigungssignalen erhält, das Signal in dem Frequenzbereich in Kurzzeitfenstern (60) analysiert und die mittlere Tiefe ($s_m$) von Brustkompressionen in jedem Fenster (60) anhand der mittleren Frequenz ($f_{cc}$) und der Amplituden und Phasen der ersten Harmonischen des Signals jedes Fensters (60) schätzt.

2. Vorrichtung nach dem vorstehenden Anspruch, wobei die Beschleunigungssensoreinheit (11) ein Ein-, Zwei- oder Dreiachsen-Beschleunigungsmesser oder ein, zwei oder drei Einachsen-Beschleunigungsmesser sind, die orthogonal zueinander angeordnet sind, wobei das Verarbeitungsmittel (21) das Beschleunigungssignal senkrecht zur Brust (3) des Patienten oder Modells (2) als Kombination der durch die Beschleunigungssensoreinheit (11) erzeugten Beschleunigungssignale ermittelt.

3. Vorrichtung nach Anspruch 1 oder 2, wobei das Kompressionskissen (10) ein flexibler Träger ist, der ein Biegen und/oder Verdrehen ermöglicht, wobei die Beschleunigungssensoreinheit (11) innerhalb des Kompressionskissens (10) angeordnet ist, wobei die Beschleunigungssensoreinheit (11) vorzugsweise das einzige Element der Einheit (100) das, das im Kompressionskissen (10) angeordnet ist.

4. Vorrichtung nach einem der Ansprüche 1 bis 3, die ein Verarbeitungs- und Feedbackmodul (20) umfasst, wobei das Verarbeitungsmittel (21), das Feedbackmittel (22) angeordnet sind, um Feedback über die Abweichungen der mittleren Tiefe ($s_m$) und der mittleren Frequenz ($f_{cc}$) von Brustkompressionen in Bezug auf empfohlene Referenzwerte visuell oder akustisch an den HLW-Assistenten bereitzustellen, und ein Energieversorgungsmittel (23) angeordnet ist, um die unterschiedlichen Mittel der Vorrichtung (100) mit Energie zu versorgen, wobei das Kompressionskissen (10) und das Verarbeitungs- und Feedbackmodul (20) elektrisch verbunden sind.

5. Vorrichtung nach Anspruch 4, wobei das Verarbeitungs- und Rückkopplungsmodul (20) ein Datenspeichermittel (24) zum Speichern der Werte der mittleren Tiefe ($s_m$) und der mittleren Frequenz ($f_{cc}$) von Brustkompressionen und der Parameter umfasst, die während der Analyse der Beschleunigungssignale im Frequenzbereich vom Verarbeitungsmittel (21) berechnet werden, wobei dieses Datenspeichermittel (24) fest oder abnehmbar ist.

6. Vorrichtung nach Anspruch 4 oder 5, wobei das Verarbeitungs- und Feedbackmodul (20) ein Kommunikationsmittel (25) zum kabelgebundenen oder drahtlosen Austausch von Informationen mit externen Vorrichtungen umfasst.

7. Vorrichtung nach einem der Ansprüche 4 bis 6, wobei das Kompressionskissen (10) und das Verarbeitungs- und Feedbackmodul (20) mechanisch miteinander verbunden sind, wobei die Vorrichtung (100) auf der Brust (3) des Patienten oder Modells (2) platziert ist, wobei die Hände des Assistenten (1) auf dem Kompressionskissen (10) platziert sind oder das Kompressionskissen (10) zwischen den Händen des Assistenten (1) angeordnet sind.

8. Vorrichtung nach einem der Ansprüche 4 bis 6, wobei das Kompressionskissen (10) und das Verarbeitungs- und Feedbackmodul (20) über ein Kabel (30) mit Verbindern (31) an jedem der Enden davon elektrisch verbunden sind, wobei das Kabel (30) eine Sammlertrommel (40) aufweist, die das Platzieren des Verarbeitungs- und Feedbackmoduls (20) in unterschiedlichen Positionen ermöglicht, wobei das Kompressionskissen (10) auf der Brust (3) des Patienten oder Modells (2) platziert ist, wobei die Hände des Assistenten (1) auf dem Kompressionskissen (10) platziert sind oder das Kompressionskissen (10) zwischen den Händen des Assistenten (1) platziert ist.

9. Vorrichtung nach einem der Ansprüche 4 bis 6, wobei das Kompressionskissen (10) und das Verarbeitungs- und Feedbackmodul (20) durch ein Fixierungsmittel (50) mechanisch miteinander verbunden sind, das das Befestigen des Verarbeitungs- und Feedbackmoduls (20) am Rücken einer der Hände des Assistenten (1) ermöglicht, wobei das Fixierungsmittel (50) eine elektrische Verbindung zwischen dem Kompressionskissen (10) und dem Verarbeitungs- und Feedbackmodul (20) ermöglicht, wobei das Kompressionskissen (10) auf der Brust (3) des Patienten oder Modells (2) platziert ist, wobei die Hände des Assistenten (1) auf dem Kompressionskissen (10) platziert sind oder das Kompressionskissen (10) zwischen den Händen des Assistenten (1) platziert ist.

10. Vorrichtung nach einem der Ansprüche 4 bis 6, wobei das Kompressionskissen (10) und das Verarbeitungs- und Feedbackmodul (20) über ein Kabel (30) mit Verbindern (31) elektrisch verbunden sind, wobei das Verarbeitungs- und Feedbackmodul (20) in eine externe Vorrichtung (200) integriert ist, und wobei das Kompressionskissen (10) auf der Brust (3) des Patienten oder Modells (2) platziert ist, wobei die Hände des Assistenten (1) auf dem Kompressionskissen (10) platziert sind oder das Kompressionskissen (10) zwischen den Händen des Assistenten (1) platziert ist.

11. Verfahren zur Unterstützung bei der Durchführung von Brustkompressionen an einem Patienten oder Modell (2) während einer Herz-Lungen-Wiederbelebung (HLW), **dadurch gekennzeichnet, dass** ein Signal, das für die Bewegung der Brust (3) des Patienten oder Modells (2) repräsentativ ist, vorzugsweise ein Beschleunigungssignal senkrecht zur Brust (3) des Patienten oder Modells (2), im Frequenzbereich von Kurzzeitfenstern (60) analysiert wird, und dass die mittlere Tiefe ($s_m$) von Brustkompressionen in jedem Fenster (60) anhand der mittleren Frequenz ($f_{cc}$) und der Amplituden und Phasen der ersten Harmonischen des Signals jedes Fensters (60) geschätzt wird.

12. Verfahren nach Anspruch 11, das in einer Vorrichtung (100) umgesetzt wird, die sich für die Unterstützung bei der Durchführung von Brustkompressionen an einem Patienten oder Modell (2) während einer HLW eignet, die ein Kompressionskissen (10), wobei der HLW-Assistent (1) manuell Kraft ausübt, um Brustkompressionen durchzuführen, wobei das Kompressionskissen (10) intern eine Beschleunigungssensoreinheit (11) umfasst, die Beschleunigungssignale erzeugt, die die Bewegung der Brust (3) des Patienten oder Modells (2) während Brustkompressionen anzeigt, ein Verarbeitungsmittel (21) zum Verarbeiten der von der Beschleunigungssensoreinheit (11) empfangenen Beschleunigungssignale und ein Feedbackmittel (22) zum Bereitstellen von Feedback an den Assistenten (1) umfasst, wobei das Verfahren umfasst:

- einen Brustkompressionsschritt (S1) zum Komprimieren der Brust eines Patienten, wobei das Verarbeitungsmittel (21) die Beschleunigungssignale von der Beschleunigungssensoreinheit (11) empfängt und ein Beschleunigungssignal senkrecht zur Brust (3) des Patienten oder Modells (2) als Kombination der von der Beschleunigungssensoreinheit (11) erzeugten Beschleunigungssignale ermittelt,
- einen Verarbeitungsschritt (S2) zum Verarbeiten des Beschleunigungssignals senkrecht zur Brust (3) des Patienten oder Modells (2), wobei das Verarbeitungsmittel (21) den Wert der mittleren Frequenz ($f_{cc}$) und der mittleren Tiefe ($s_m$) von Brustkompressionen in jedem ausgewählten Fenster (60) berechnet, und
- einen Feedbackschritt (S3) zum Bereitstellen von Feedback an den Assistenten (1), wobei das Feedbackmittel (22) die Abweichungen der mittleren Tiefe ($s_m$) und der Frequenz ($f_{cc}$) von Brustkompressionen in Bezug auf Referenzwerte als Feedback an den Assistenten (1) bereitstellt.

13. Verfahren nach Anspruch 12, wobei der Verarbeitungsschritt (S2) umfasst:

- eine Fensterungsphase (P1) zum Fenstern des Beschleunigungssignals senkrecht zur Brust (3) des Patienten oder Modells (2), wobei das Verarbeitungsmittel (21) die Kurzzeitfenster (60) für das empfangene Beschleunigungssignal auswählt,
- eine Spektralanalysephase (P2) nach der Fensterungsphase (P1), wobei das Verarbeitungsmittel (21) eine Spektralanalyse der Fenster (60) des Beschleunigungssignals im Frequenzbereich durchführt, das Frequenzspektrum (70) des Beschleunigungssignals durch einen Frequenzschätzer berechnet, im Frequenzspektrum (70) des Beschleunigungssignals die Basisfrequenz oder den Wert der mittleren Frequenz ($f_{cc}$) von Brustkompressionen in jedem ausgewählten Fenster (60) und die Parameter $A_i$ und $\theta_i$ berechnet, die jeweils der Wert der Amplitude und der Phase der Harmonischen des Frequenzspektrums des Beschleunigungssignals der Größenordnung i jedes Fensters (60) für eine Reihe von Harmonischen i = 1, 2 ... N ist, die für das Frequenzspektrum (70) repräsentativ sind,
- eine Berechnungsphase (P3) zum Berechnen der Werte der Tiefenparameter jedes ausgewählten Fensters (60) nach der Spektralanalysephase (P2), wobei das Verarbeitungsmittel (21) die Werte der Tiefenparameter $S_i$ und $\psi_i$, die jeweils die Werte der Amplitude und der Phase der Harmonischen des Tiefenspektrums der Größenordnung i sind, anhand der Formeln

$$S_i = 1000 \times Ai / 2\pi \times i \times fcc)^2,$$

$$\Psi_i = \theta_i + \pi$$

 für eine Reihe von Harmonischen i = 1, 2 ... N berechnet, die für das Tiefenspektrum repräsentativ sind, und
- eine Schätzphase (P4) zum Schätzen der mittleren Tiefe ($s_m$) von Brustkompressionen jedes Fensters (60) nach der Berechnungsphase (P3) zum Berechnen der Werte der Tiefenparameter, wobei das Verarbeitungsmittel (21) den Wert der mittleren Tiefe ($s_m$) anhand des Werts von Parametern $S_i$ und $\psi_i$ unter Verwendung eines Schätzers berechnet.

14. Verfahren nach Anspruch 12 oder 13, wobei die Vorrichtung (100) ein Datenspeichermittel (24) und Kommunikationsmittel (25) umfasst, wobei das Verfahren einen Datenspeicher- und Kommunikationsschritt (S4) nach dem Berichterstattungsschritt (S3) umfasst, wobei die Werte der mittleren Frequenz ($f_{cc}$) und der mittleren Tiefe ($s_m$) von Brustkompressionen sowie die Werte von Parametern $A_i$, $\theta_i$, $S_i$ und $\psi_i$ einer Reihe von Harmonischen i = 1, 2 ... N jedes ausgewählten Fensters (60) im Datenspeichermittel (24) gespeichert und/oder vom Kommunikationsmittel (25) an eine externe Vorrichtung (200) gesendet werden.

15. Verfahren nach Anspruch 13 oder 14, das im Verarbeitungsschritt (S2) eine Signalkonditionierungsphase (P0) vor der Fensterungsphase (P1), in der das vom Verarbeitungsmittel (21) empfangene Beschleunigungssignal senkrecht zur Brust (3) des Patienten oder Modells (2) mithilfe eines Filterungssystems konditioniert wird, das die DC-Komponente oder Niederfrequenzkomponenten des Beschleunigungssignals eliminiert, und eine Verifizierungsphase (P11) zum Verifizieren des Vorhandenseins von Brustkompressionen nach der Fensterungsphase (P1) umfasst, wobei das Beschleunigungssignal analysiert wird, um das Vorhandensein von Brustkompressionen in jedem Fenster (60) zu verifizieren, und die Entscheidung zur Fortsetzung mit der Spektralanalysephase getroffen wird, wenn Brustkompressionen im Fenster (60) durchgeführt werden, oder wenn Brustkompressionen im Fenster (60) nicht durchgeführt werden, auf das Einlangen des darauf folgenden Fensters (60) des Beschleunigungssignals gewartet sind und die Verifizierungsphase (P11) zum Verifizieren des Vorhandenseins von Brustkompressionen neu gestartet wird.

## Revendications

1.  Dispositif destiné à faciliter l'exécution de compressions thoraciques sur un patient ou un mannequin (2) pendant une réanimation cardiorespiratoire (CPR) qui comprend un coussin de compression (10) prévu pour suivre le mouvement du thorax (3) du patient ou du mannequin (2), dans lequel l'assistant de réanimation cardiorespiratoire (1) exerce manuellement une force destinée à effectuer des compressions thoraciques, ledit coussin de compression (10) comprenant un capteur d'accélération (11) qui produit des signaux d'accélération représentatifs du mouvement du thorax (3) du patient ou du mannequin (2) pendant les compressions thoraciques, et le dispositif (100) comprenant également un moyen de traitement (21) destiné à déterminer la profondeur des compressions thoraciques à partir des signaux d'accélération reçus de la part du capteur d'accélération (11), **caractérisé en ce que** le moyen de traitement (21) obtient un signal représentatif du mouvement du thorax (3) du patient ou du mannequin (2), de préférence un signal d'accélération perpendiculaire au thorax (3) du patient ou du mannequin (2), à partir desdits signaux d'accélération, analyse ledit signal dans le domaine de fréquences dans des fenêtres de laps de temps court (60), et estime la profondeur moyenne ($s_m$) des compressions thoraciques dans chaque fenêtre (60) à partir de la fréquence moyenne ($f_{cc}$) et des amplitudes et des phases du premier harmonique du signal de chaque fenêtre (60).

2.  Dispositif selon la revendication précédente, dans lequel le capteur d'accélération (11) est un accéléromètre à axe simple, double ou triple, ou un, deux ou trois accéléromètres à axe simple agencés de manière orthogonale les uns par rapport aux autres, le moyen de traitement (21) déterminant le signal d'accélération perpendiculaire au thorax (3) du patient ou du mannequin (2) sous forme de combinaison des signaux d'accélération produits par le capteur d'accélération (11).

3.  Dispositif selon la revendication 1 ou 2, dans lequel le coussin de compression (10) est un support flexible qui permet une flexion et/ou une torsion, le capteur d'accélération (11) étant placé à l'intérieur dudit coussin de compression (10), et ledit capteur d'accélération (11) étant de préférence le seul élément du dispositif (100) placé dans le coussin de compression (10).

4.  Dispositif selon l'une quelconque des revendications 1 à 3, qui comprend un module de traitement et de retour (20) dans lequel sont placés le moyen de traitement (21), un moyen de retour (22) destiné à fournir un retour à l'assistant de réanimation cardiorespiratoire, visuellement ou de manière sonore, à propos des écarts de la profondeur moyenne ($s_m$) et de la fréquence moyenne ($f_{cc}$) des compressions thoraciques par rapport aux valeurs de référence recommandées, et un moyen d'alimentation en énergie (23) destiné à alimenter les différents moyens du dispositif (100), le coussin de compression (10) et le module de traitement et de retour (20) étant reliés électriquement.

5.  Dispositif selon la revendication 4, dans lequel le module de traitement et de retour (20) comprend un moyen de stockage de données (24) destiné à enregistrer les valeurs de la profondeur moyenne ($s_m$) et de la fréquence moyenne ($f_{cc}$) des compressions thoraciques et les paramètres calculés pendant l'analyse des signaux d'accélération dans le domaine de fréquences par le moyen de traitement (21), ce moyen de stockage de données (24) étant fixe ou amovible.

6.  Dispositif selon la revendication 4 ou 5, dans lequel le module de traitement et de retour (20) comprend un moyen de communication (25) destiné à échanger des informations avec des dispositifs externes, de manière sans fil ou câblée.

7.  Dispositif selon l'une quelconque des revendications 4 à 6, dans lequel le coussin de compression (10) et le module de traitement et de retour (20) sont mécaniquement reliés l'un à l'autre, le dispositif (100) étant placé sur le thorax (3) du patient ou du mannequin (2) avec les mains de l'assistant (1) placées sur le coussin de compression (10), ou le coussin de compression (10) étant situé entre les mains de l'assistant (1).

8.  Dispositif selon l'une quelconque des revendications 4 à 6, dans lequel le coussin de compression (10) et le module de traitement et de retour (20) sont reliés électriquement à l'aide d'un câble (30) muni de connecteurs (31) à chacune de ses extrémités, ledit câble (30) ayant une bobine collectrice (40) qui permet de placer le module de traitement et de retour (20) dans différentes positions, le coussin de compression (10) étant placé sur le thorax (3) du patient ou du mannequin (2) avec les mains de l'assistant (1) placées sur le coussin de compression (10), ou le coussin de compression (10) étant placé entre les mains de l'assistant (1).

9. Dispositif selon l'une quelconque des revendications 4 à 6, dans lequel le coussin de compression (10) et le module de traitement et de retour (20) sont mécaniquement reliés l'un à l'autre par un moyen de fixation (50) qui permet de fixer le module de traitement et de retour (20) sur l'arrière de l'une des mains de l'assistant (1), ledit moyen de fixation (50) permettant la liaison électrique entre le coussin de compression (10) et le module de traitement et de retour (20), le coussin de compression (10) étant placé sur le thorax (3) du patient ou du mannequin (2) avec les mains de l'assistant (1) placées sur le coussin de compression (10), ou le coussin de compression (10) étant placé entre les mains de l'assistant (1).

10. Dispositif selon l'une quelconque des revendications 4 à 6, dans lequel le coussin de compression (10) et le module de traitement et de retour (20) sont reliés électriquement à l'aide d'un câble (30) muni de connecteurs (31), le module de traitement et de retour (20) étant intégré à un dispositif externe (200), et le coussin de compression (10) étant placé sur le thorax (3) du patient ou du mannequin (2) avec les mains de l'assistant (1) placées sur le coussin de compression (10), ou le coussin de compression (10) étant placé entre les mains de l'assistant (1).

11. Procédé destiné à faciliter l'exécution de compressions thoraciques sur un patient ou un mannequin (2) pendant une réanimation cardiorespiratoire (CPR), **caractérisé en ce qu'**un signal représentatif du mouvement du thorax (3) du patient ou du mannequin (2), de préférence un signal d'accélération perpendiculaire au thorax (3) du patient ou du mannequin (2), est analysé dans le domaine de fréquences dans des fenêtres de laps de temps court (60), et la profondeur moyenne ($s_m$) des compressions thoraciques dans chaque fenêtre (60) est estimée à partir de la fréquence moyenne ($f_{cc}$) et des amplitudes et des phases du premier harmonique du signal de chaque fenêtre (60).

12. Procédé selon la revendication 11, mis en oeuvre dans un dispositif (100) adapté pour faciliter l'exécution de compressions thoraciques sur un patient ou un mannequin (2) pendant une réanimation cardiorespiratoire, qui comprend un coussin de compression (10), dans lequel l'assistant de réanimation cardiorespiratoire (1) exerce manuellement une force destinée à effectuer des compressions thoraciques, ledit coussin de compression (10) comprenant un capteur d'accélération (11) qui produit des signaux d'accélération représentatifs du mouvement du thorax (3) du patient ou du mannequin (2) pendant les compressions thoraciques, un moyen de traitement (21) destiné à traiter les signaux d'accélération reçus de la part du capteur d'accélération (11), et un moyen de retour (22) destiné à fournir un retour à l'assistant (1), ledit procédé comprenant :

- une étape de compression thoracique (S1) destinée à comprimer le thorax du patient, le moyen de traitement (21) recevant les signaux d'accélération de la part du capteur d'accélération (11) et déterminant un signal d'accélération perpendiculaire au thorax (3) du patient ou du mannequin (2), sous forme de combinaison des signaux d'accélération produits par le capteur d'accélération (11),
- une étape de traitement (S2) destinée à traiter le signal d'accélération perpendiculaire au thorax (3) du patient ou du mannequin (2), le moyen de traitement (21) calculant la valeur de la fréquence moyenne ($f_{cc}$) et de la profondeur moyenne ($s_m$) des compressions thoraciques dans chaque fenêtre sélectionnée (60), et
- une étape de retour (S3) destinée à fournir un retour à l'assistant (1), le moyen de retour (22) fournissant en retour à l'assistant (1) les écarts de profondeur moyenne ($s_m$) et de fréquence ($f_{cc}$) des compressions thoraciques par rapport aux valeurs de référence recommandées.

13. Procédé selon la revendication 12, dans lequel l'étape de traitement (S2) comprend :

- une phase de fenêtrage (P1) destinée à fenêtrer le signal d'accélération perpendiculaire au thorax (3) du patient ou du mannequin (2), le moyen de traitement (21) choisissant les fenêtres de laps de temps court (60) pour le signal d'accélération reçu,
- une phase d'analyse spectrale (P2) après la phase de fenêtrage (P1), le moyen de traitement (21) effectuant une analyse spectrale des fenêtres (60) du signal d'accélération dans le domaine de fréquences, calculant le spectre de fréquences (70) du signal d'accélération à l'aide d'un estimateur de fréquence, calculant, dans le spectre de fréquences (70) du signal d'accélération, la fréquence fondamentale ou la valeur de la fréquence moyenne ($f_{cc}$) des compressions thoraciques dans chaque fenêtre sélectionnée (60), et les paramètres $A_i$ et $\theta_i$, qui sont la valeur de l'amplitude et de la phase de l'harmonique du spectre de fréquences du signal d'accélération de l'ordre i, respectivement, de chaque fenêtre (60) pur un nombre d'harmoniques i = 1, 2, ..., N, représentatif du spectre de fréquences (70),

- une phase de calcul (P3) destinée à calculer les valeurs des paramètres de profondeur de chaque fenêtre sélectionnée (60) après la phase d'analyse spectrale (P2), le moyen de traitement (21) calculant les valeurs des paramètres de profondeur Si et $\psi_i$, qui sont la valeur de l'amplitude et de la phase de l'harmonique du spectre de profondeurs de l'ordre i, respectivement, à l'aide des formules

$$S_i = 1000 \times Ai / 2\pi \times i \times fcc)^2, \text{ et}$$

$$\Psi_i = \theta_i + \pi$$

pour un nombre d'harmoniques i = 1, 2, ..., N représentatif du spectre de profondeurs, et

- une phase d'estimation (P4) destinée à estimer la profondeur moyenne ($s_m$) des compressions thoraciques de chaque fenêtre (60) après la phase de calcul (P3) afin de calculer les valeurs des paramètres de profondeur, le moyen de traitement (21) calculant la valeur de la profondeur moyenne ($s_m$) à partir des valeurs des paramètres $S_i$ et $\psi_i$, en utilisant un estimateur.

14. Procédé selon la revendication 12 ou 13, dans lequel le dispositif (100) comprend un moyen de stockage de données (24) et un moyen de communication (25), le procédé comprenant une étape de communication et de stockage de données (S4) après l'étape de reporting (S3), dans lequel les valeurs de la fréquence moyenne ($f_{cc}$) et de la profondeur moyenne ($s_m$) des compressions thoraciques, et les valeurs des paramètres $A_i$, $\theta_i$, $S_i$ et $\psi_i$ d'un d'un nombre d'harmoniques i = 1, 2, ..., N, de chaque fenêtre sélectionnée (60), sont enregistrées dans le moyen de stockage de données (24) et/ou envoyées à un dispositif externe (200) à l'aide du moyen de communication (25).

15. Procédé selon la revendication 13 ou 14, qui comprend, à l'étape de traitement (S2), une phase de conditionnement de signal (P0) avant la phase de fenêtrage (P1), pendant laquelle le signal d'accélération perpendiculaire au thorax (3) du patient ou du mannequin (2) reçu par le moyen de traitement (21) est conditionné à l'aide d'un système de filtrage qui élimine la composante CC ou les composantes à basse fréquence du signal d'accélération, et une phase de vérification (P11) destinée à vérifier l'existence de compressions thoraciques après la phase de fenêtrage (P1), au cours de laquelle le signal d'accélération est analysé afin de vérifier l'existence de compressions thoraciques dans chaque fenêtre (60), et dans lequel la décision de continuer avec la phase d'analyse spectrale est prise si des compressions thoraciques ont lieu dans la fenêtre (60), ou, si aucune compression thoracique n'a lieu dans la fenêtre (60), l'arrivée de la fenêtre suivante (60) du signal d'accélération est attendue, et la phase de vérification (P11) destinée à vérifier l'existence des compressions thoraciques est relancée.

**FIG. 1**

**FIG. 2**

**FIG. 3**

**FIG. 4**

**FIG. 5**

**FIG. 6**

**FIG. 7**

**FIG. 8**

FIG. 9

FIG. 10

FIG. 11

FIG. 12

**FIG. 13**

FIG. 14

FIG. 15a

FIG. 15b

FIG. 15c

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1057451 A1